# EUROPÄISCHE PATENTSCHRIFT

(11) **EP 1 891 908 B1**
(45) Veröffentlichungstag und Bekanntmachung des Hinweises auf die Patenterteilung: **25.02.2009**
(21) Anmeldenummer: 06017395.2
(22) Anmeldetag: 22.08.2006
(51) Int. Cl.: A61B 19/00, A61B 17/00

(54) **Trackingfähiges medizinisches Instrument mit austauschbarer Spitze**
Trackable medical instrument with replaceable tip
Instrument chirurgical repérable avec extrémité remplaçable

(43) Veröffentlichungstag der Anmeldung: 27.02.2008
(73) Patentinhaber: BrainLAB AG, 85622 Feldkirchen (DE)
(72) Erfinder: Stumpf, Tanja, 80799 München (DE)
(74) Vertreter: Schwabe - Sandmair - Marx

(56) Entgegenhaltungen:
- EP-A1- 1 543 789
- DE-A1- 19 639 615
- DE-U1- 29 600 990
- US-A- 5 617 857
- US-A1- 2002 193 800
- US-A1- 2005 113 659

## Beschreibung

Die Erfindung betrifft ein trackingfähiges medizinisches Instrument mit einer austauschbaren Spitze. Der Gattung nach betrifft die Erfindung ein medizinisches Instrument mit einem Griff und einem funktionellen Abschnitt, wobei an dem Instrument eine Anordnung von Trackingmarkern angeordnet ist. Solche Instrumente sind für den Einsatz in der bildunterstützten Chirurgie vorgesehen, und die Trackingmarker dienen zum Tracking des Instruments mittels eines medizintechnischen Trackingsystems und zur Identifizierung des Instruments durch ein medizintechnisches Navigationssystem. Mit Hilfe des Navigationssystems kann die Lage des Instruments, insbesondere seines funktionellen Abschnittes (Spitze) geortet und verfolgt und das Instrument beispielsweise in räumlicher Relation zu vorab ermittelten Patientendaten (aus bildgebenden Verfahren; CT, MRI, etc.) auf einer Bildausgabe (Bildschirm) dargestellt werden, um dem Chirurgen bei seiner Arbeit eine visuelle Unterstützung zu geben.

Medizinische Instrumente mit Trackingmarkern oder Trackingmarker-Aufsätzen sind bekannt, beispielsweise aus der DE 196 39 615 A1.

Um solche Instrumente, z. B. Instrumente mit Reflektorenmarker-Anordnungen, eindeutig durch ein Navigationssystem identifizierbar machen zu können, wird gemäß dem Stand der Technik jedem der Instrumente eine Referenzmarkergruppe zugeordnet, die eine für dieses Instrument charakteristische Anordnung aufweist. Dadurch wird die Herstellung solcher Instrumente inklusive der Bestückung mit Referenzmarkergruppen aufwendig und teuer, insbesondere wenn sie als Instrumentensatz zur Verfügung gestellt werden.

Es ist die Aufgabe der vorliegenden Erfindung ein medizinisches Instrument bzw. einen Satz medizinischer Instrumente zur Verfügung zu stellen, welche die oben genannten Nachteile des Standes der Technik überwinden.

Diese Aufgabe wird durch ein medizinisches Instrument gemäß dem Patentanspruch 1 sowie durch einen medizinischen Instrumentensatz gemäß dem Patentanspruch 9 gelöst. Die Unteransprüche definieren bevorzugte Ausführungsformen der Erfindung.

Erfindungsgemäß ist ein medizinisches Instrument in mindestens zwei Teile teilbar und wieder zusammensetzbar, wobei an jedem Teil mindestens ein Trackingmarker angeordnet ist. Diese Teilbarkeit ergibt eine Austauschbarkeit eines Instrumententeils, und damit ergibt sich die Möglichkeit, die Anordnung der Trackingmarker des Instruments durch den Austausch von Instrumententeilen zu beeinflussen. Auf einem Teil des Instruments können dann ein oder mehrere Trackingmarker so verbleiben, wie sie sind, und wenn ein Austausch-Instrumententeil mit dem verbleibenden Instrumententeil kombiniert wird, entsteht eine neue Markeranordnung, die wiederum charakteristisch für das neue Instrument ist. Mit der Erfindung erübrigt sich somit die Notwendigkeit, vollständige neue Markeranordnungen für unterschiedliche Instrumente bereitzustellen.

Bei einer Ausführungsform der vorliegenden Erfindung umfasst ein erster Teil des Instruments den funktionellen Abschnitt, also beispielsweise die Instrumentenspitze, die je nach Instrument unterschiedlich ausgestaltet und für unterschiedliche Arbeitsvorgänge geeignet sein kann (zum Beispiel Pointerspitze, Pinzette, Skalpellklinge), und ein zweiter Teil des Instruments umfasst mindestens einen Teil des Griffes (das Instrument ist also im Griff teilbar). Dabei besteht die Möglichkeit, am ersten Teil des Instruments mindestens einen Trackingmarker oder eine Trackingmarkergruppe anzuordnen, wobei der Trackingmarker oder die Trackingmarkergruppe in ihrer Ausgestaltung oder Anordnung eindeutig dem funktionellen Abschnitt des Instruments zugeordnet ist. Damit ergibt sich für jeden ersten Teil des Instruments, mit dem funktionellen Abschnitt, eine separate und unterscheidbare Gesamt-Trackingmarkeranordnung am Instrument. Die Gesamtgeometrie des Trackingmarkers oder der Trackingmarkergruppe des funktionellen Abschnittes zusammen mit dem Trackingmarker oder der Trackingmarkergruppe des Distalteils sind charakteristisch für eine Instrumentenzusammensetzung und dienen derer individuellen Erkennung. Die individuelle Erkennbarkeit ist gewährleistet, wenn für gleiche Geometrien des Distalteils die Postion des Trackingmarkers bzw. die Positionen der Trackingmarkergruppe sich zumindest in der Axialposition unterscheiden, generell kann eine Änderung der Axial- sowie der Radialposition für die Variabilität der Geometrien genutzt werden.

Bei den aufgezeigten medizinischen Instrumenten besteht die Möglichkeit, am ersten Instrumententeil einen Trackingmarker und am zweiten Instrumententeil einen weiteren Trackingmarker anzuordnen, während andererseits auch am ersten Instrumententeil ein Trackingmarker und am zweiten Instrumententeil eine Trackingmarkergruppe mit zwei oder mehreren Trackingmarkern angeordnet werden kann. Natürlich kann auch das erste Instrumententeil mehrere Trackingmarker aufweisen, wichtig ist lediglich, dass die oder der Trackingmarker für das erste Instrumententeil eine einzigartige Anordnung haben, die diesem Instrumententeil zuordnungsfähig ist.

Die beiden Instrumententeile sind bei einer bevorzugten Ausführungsform durch eine drehfeste Verbindung verbindbar, wobei die Verbindung insbesondere eine oder mehrere der folgenden Verbindungsarten umfasst: eine Schraubgewindeverbindung mit formschlüssigen oder reibschlüssigen Eingriffsmitteln zwischen dem ersten und zweiten Instrumententeil; eine Schnappverbindung mit formschlüssigen oder reibschlüssigen Eingriffsmitteln zwischen dem ersten und zweiten Instrumententei; eine Steckverbindung mit formschlüssigen oder reibschlüssigen Eingriffsmitteln zwischen dem ersten und zweiten Instrumententeil.

Wie am Eingang schon kurz vermerkt, können die Trackingmarker eines erfindungsgemäßen medizinischen Instruments zum Tracken des Instruments mittels eines medizintechnischen Trackingsystems und zur Identifizierung des Instruments durch ein medizintechnisches Navigationssystem dienen.

Die Erfindung umfasst ferner einen medizinischen Instrumentensatz mit mindestens zwei Instrumenten, wie sie oben in verschiedenen Ausführungen beschrieben worden sind. "zwei Instrumente" bedeutet nicht unbedingt, dass zwei erste Instrumententeile und zwei zweite Instrumententeile vorhanden sein müssen, vielmehr können die Instrumente aus verschiedenen ersten Instrumententeilen, die jeweils den funktionellen Abschnitt umfassen, und aus demselben oder aus gleichen zweiten Instrumententeilen, die mindestens einen Teil des Griffes umfassen, aufgebaut sein. Grundsätzlich genügt ein einziges zweites Instrumententeil, an dem mehrere erste Instrumententeile austauschbar angebracht werden können, um einen erfindungsgemäßen Instrumentensatz bereitzustellen.

Die Erfindung hilft einem Verwender, beispielsweise einem Chirurgen, sein Instrument an die situationsbedingten Anforderungen anzupassen. Es ist nur notwenig, den vorderen Teil eines Instruments auszutauschen, um ein völlig anderes Instrument herzustellen, wobei der neue Vorderteil (erster Instrumententeil oder Distalteil) mit seinem Referenzmarker bzw. seinen Referenzmarkern zusammen mit dem hinteren Instrumententeil (zweiter Instrumententeil oder Proximalteil) vom Navigationssystem erkannt wird, und zwar aufgrund der Konfiguration bzw. Anordnung aller am Instrument befindlichen Marker. Dazu können die verschiedenen Kombinationen im Navigationssystem hinterlegt werden. Am Erkennen einer Kombination kann dann das neu kombinierte Instrument eindeutig erkannt und zugeordnet werden, und das Navigationssystem muss nicht mehr separat darüber informiert werden, welches Instrument gerade verwendet wird.

Die Erfindung macht eine leichte Anpassung des Instruments an den tatsächlich vorhandenen Bedarf möglich. Es ist nicht mehr notwendig, eine Vielzahl unterschiedlicher vollständiger Instrumente bereitzustellen, grundsätzlich genügt ein Proximalteil (zweites Instrumententeil) und eine Sammlung mehrerer Distalteile. Die Geometrie der trackingfähigen passiven Markeranordnungen kann automatisch vom Navigationssystem erkannt werden.

Man kann die Erfindung auch so beschreiben, dass sie ein medizinisches Instrument bereitstellt, dessen Vorderteil oder Spitze austauschbar ist. Wenn beispielsweise zwei optische Trackingmarker an einem Standard-Proximalteil (hinteres oder zweites Instrumententeil) angeordnet sind, kann ein dritter an dem austauschbaren Vorderteil angeordnet werden. Die Distanz vom dritten Marker zu einem Punkt auf dem Standard-Proximalteil (z.B. einem Marker oder auch einem Teil des Instruments selbst (Stirnseite) und/oder seine Höhe über der Achse seines Standard-Proximalteils bilden eine charakteristische und einzigartige Geometrie der Trackingmarker. Eines der beiden Maße oder beide Maße zusammen (Abstand und Höhe) sind für jedes austauschbare Distalteil unterschiedlich und können deshalb eindeutig zugeordnet werden.

Die Erfindung wird im Weiteren anhand mehrer Ausführungsformen näher erläutert. Sie kann die hierin beschriebenen Merkmale einzeln sowie in jedweder sinnvollen Kombination umfassen. In den beiliegenden Zeichnungen zeigen:
- Figuren 1 und 2: ein erfindungsgemäß gestaltetes Instrument mit gerader Instrumentenspitze und jeweils zwei bzw. drei Trackingmarkern;
- Figuren 3 und 4: ein anderes erfindungsgemäß gestaltetes Instrument mit gekrümmter Spitze, mit zwei bzw. drei Trackingmarkern;
- Figuren 5 und 6: noch ein weiteres erfindungsgemäß gestaltetes Instrument, wiederum mit jeweils zwei bzw. drei Trackingmarkern;
- Figur 7: eine Verbindungsmöglichkeit für zwei Instrumententeile mit einer Schraub- und Eingriffslaschenverbindung; und
- Figur 8: eine Verbindungsmöglichkeit für zwei Instrumententeile mit einer Steck- bzw. Schnappverbindung.

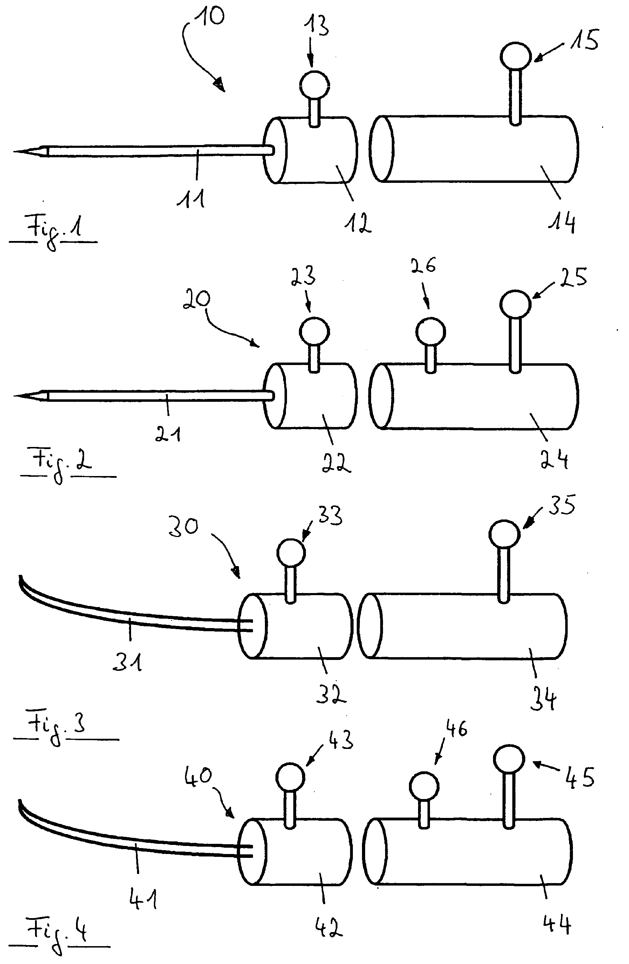

Die Figuren 1 und 2 zeigen ein erfindungsgemäß ausgestaltetes Instrument mit gleichem Distalteil, aber mit unterschiedlichen Proximalteilen. Die beiden Instrumente sind in ihrer Gesamtheit in den Figuren 1 und 2 mit den Bezugszeichen 10 und 20 angedeutet. Sie weisen gerade Instrumentenspitzen 11, 21 auf, die jeweils den vorderen Abschnitt des Distalteils 12, 22 bilden. Auf den Distalteilen sind passive Reflektionsmarker 13, 23 angebracht, und zwar in einem mittleren Abstand von der Instrumentenachse. Die Distalteile 12, 22 sind verbindbar mit Proximalteilen 14, 24, wobei das Proximalteil 14 in der Figur 1 nur einen einzigen Trackingmarker 15 aufweist. Die charakteristische Konfiguration der Markeranordnung würde im Falle des Instruments nach Figur 1 durch den Abstand zwischen den Markern 13 und 15 bestimmt. Beim Instrument 20 nach Figur 2 wird die charakteristische Anordnung durch die relativen Abstände zwischen den drei Markern 23, 25 und 26 auf dem Distalteil 22 und dem Proximalteil 24 bestimmt.

Die Instrumente sind in den Figuren geteilt dargestellt, um die Erfindung zu veranschaulichen. Natürlich sind im Verwendungszustand die Instrumententeile jeweils miteinander verbunden.

In den Figuren 3 und 4 ist ein weiteres erfindungsgemäßes Instrument gezeigt, das eine gebogene Spitze hat, die jeweils mit 31 bzw. 41 bezeichnet ist. Das Instrument kann wiederum Proximalteile 34 und 44 aufweisen, die mit den Proximalteilen 14 bzw. 24 aus den Figuren 1 und 2 identisch sind. Zur eindeutigen Identifizierung unterscheiden sich die Instrumente 30, 40 aus den Figuren 3 und 4 hiervon aber in Hinsicht auf die Anordnung des Trackingmarkers 33, 43 am Distalteil 32 bzw. 42. Es ist erkennbar, dass die Marker 33, 43 einen größeren Abstand zur Instrumentenlängsachse und zu den Markern des Distalteils haben als die Marker 13, 23, und durch dieses unterschiedliche Maß werden die Markerkonfigurationen insgesamt (Marker am Proximalteil plus Marker am Distalteil) von anderen Markerkonfigurationen (z.B. für die Instrumente 10 und 20) unterscheidbar. Aufgrund dieser unterschiedlichen Markerkonfigurationen kann ein Navigationssystem dann erkennen, dass es sich gerade um die Instrumente 30 bzw. 40 handelt, obwohl die Proximalteile 14 und 24 den Proximalteilen 34 und 44 jeweils entsprechen.

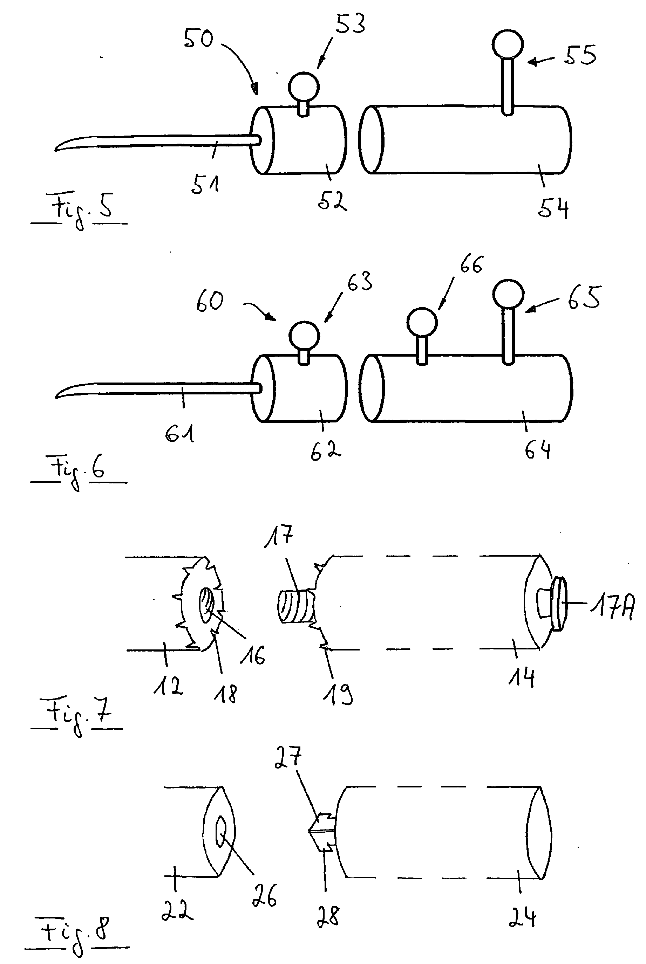

Für die Figuren 5 und 6 und die dort dargestellten Instrumente 50 und 60 gilt entsprechendes, wobei die Bezugszeichen mit entsprechenden Endziffern auch entsprechende Teile wie in den Figuren 1 bis 4 bezeichnen. In den Figuren 5 und 6 ist erkennbar, dass für das dort dargestellte Instrument mit identischen Distalteilen 52 und 62 und mit kurz gekrümmter Spitze 51 bzw. 61 der jeweilige Marker 53 bzw. 63 nur einen sehr kurzen Abstand von der Längsachse des Instruments besitzt. Aufgrund dieses kurzen Abstandes und der so veränderten Gesamt-Markerkonfiguration für das Instrument wird dieses wiederum eindeutig erkennbar und identifizierbar.

Es ist demnach z.B. möglich das Proximalteil 14 mit einem der Distalteile 11, 31, 51 zu kombinieren und jeweils ein anderes, eindeutig identifizierbares Instrument zu erhalten.

Eine Art und Weise, wie ein Distalteil 12 mit einem Proximalteil 14 verbunden werden kann, ist in der Figur 7 dargestellt. Der Distalteil 12 weist eine zentrische Gewindebohrung 16 und kerbenartige Ausnehmungen am Umriss seiner Stirnseite auf. Hierzu passend weist der Proximalteil 14 eine in ihm verdrehbare Gewindestange 17 auf, die mit dem Angriff 17A verbunden und durch diesen drehbar ist. An dem Ende, wo die Gewindestange aus dem Proximalteil 14 heraustritt, sind an dessen Umfang abstehende Laschen 19 vorgesehen. Beim Zusammenfügen der beiden Teile 12 und 14 wird die Gewindestange 17 in das Innengewinde 16 eingeschraubt, und die Laschen 19 kommen in den Kerben 18 zu liegen, so dass beim Festschrauben mittels des Angriffs 17A am Ende eine drehgesicherte Verbindung entsteht, die sicherstellt, dass die Marker in der richtigen, das heißt im Navigationssystem gespeicherten Anordnung verbleiben.

Die Figur 8 zeigt für die Ausführungsform nach Figur 2 eine weitere Verbindungsmöglichkeit, bei der der Distalteil 22 eine zentrische Bohrung 26 umfasst, in die ein Rastfortsatz 27 des Proximalteils 24 einrasten kann. Die nach außen gerichteten Rippen 28 verhindern dabei eine Drehung der Teile 22 und 24 gegeneinander, und ebenfalls ein Lösen dieser beiden Teile voneinander. Es könnte noch eine Vorrichtung vorgesehen sein, die beispielsweise die Rippen 28 wegklappt, damit das gewollte Trennen der beiden Teile voneinander in einfacher Weise geschehen kann.

Bei jeder möglichen Verbindung ist es noch von Vorteil, wenn eine Ausrichtungshilfe, beispielsweise ein mechanischer Eingriff oder eine Kennzeichnung vorgesehen wird, damit die Verbindung in der richtigen Drehstellung der beiden Teile zueinander vonstatten gehen kann.

## Patentansprüche

1. Medizinisches Instrument (10 - 60) mit einem Griff und einem funktionellen Abschnitt (11 - 61), wobei an dem Instrument eine Anordnung von Trackingmarkern (13, 14, 15 - 63, 64, 65) angeordnet ist, **dadurch gekennzeichnet, dass** das Instrument in mindestens zwei Teile teilbar und wieder zusammensetzbar ist, wobei an jedem Teil mindestens ein Trackingmarker angeordnet ist.

2. Medizinisches Instrument (10 - 60) nach Anspruch 1, **dadurch gekennzeichnet, dass** ein erster Teil (12 - 62) des Instruments (10 - 60) den funktionellen Abschnitt (11 - 61) umfasst und ein zweiter Teil (14 - 64) des Instruments (10 - 60) mindestens einen Teil des Griffes umfasst.

3. Medizinisches Instrument nach Anspruch 2, **dadurch gekennzeichnet, dass** am ersten Teil des Instruments (10 - 60) mindestens ein Trackingmarker (13 - 63) oder eine Trackingmarkergruppe angeordnet ist, wobei der Trackingmarker (13 - 63) oder die Trackingmarkergruppe in ihrer Ausgestaltung oder Anordnung eindeutig dem funktionellen Abschnitt (11 - 61) des Instruments (10 - 60) zugeordnet ist.

4. Medizinisches Instrument (10 - 60) nach Anspruch 3, **dadurch gekennzeichnet, dass** die Zuordnung des Trackingmarkers (13 - 63) oder der Trackingmarkergruppe durch eine dem funktionellen Teil zugeordnete charakteristische Anordnung, insbesondere eine Axial- oder Radialposition am ersten Instrumententeil (12 - 62) gegenüber der Trackergeometrie des zweiten Instrumententeils (14 - 64), bestimmt wird.

5. Medizinisches Instrument (10 - 60) nach einem der Ansprüche 1 bis 4, **dadurch gekennzeichnet, dass** am ersten Instrumententeil (12 - 62) ein Trackingmarker (13 - 63) und am zweiten Instrumententeil (14 -64) ein Trackingmarker angeordnet ist.

6. Medizinisches Instrument (10 - 60) nach einem der Ansprüche 1 bis 4, **dadurch gekennzeichnet, dass** am ersten Instrumententeil (12 - 62) ein Trackingmarker (13 - 63) angeordnet ist und am zweiten Instrumententeil (14 - 64) eine Trackingmarkergruppe mit zwei oder mehreren Trackingmarkern angeordnet ist.

7. Medizinisches Instrument (10 - 60) nach einem der Ansprüche 1 bis 6, **dadurch gekennzeichnet, dass** die beiden Instrumententeile (12 - 62; 14 - 64) durch eine drehfeste Verbindung verbindbar sind, wobei die Verbindung insbesondere eine oder mehrere der folgenden Verbindungsarten umfasst:
- eine Schraubgewindeverbindung (16, 17) mit formschlüssigen oder reibschlüssigen Eingriffsmitteln (18) zwischen dem ersten und zweiten Instrumententeil (12 - 62; 14 - 64);
- eine Schnappverbindung (26, 28) mit formschlüssigen oder reibschlüssigen Eingriffsmitteln (28) zwischen dem ersten und zweiten Instrumententeil (12 - 62; 14 - 64);
- eine Steckverbindung mit formschlüssigen oder reibschlüssigen Eingriffsmitteln (28) zwischen dem ersten und zweiten Instrumententeil (12 - 62; 14 - 64).

8. Medizinisches Instrument (10 - 60) nach einem der Ansprüche 1 bis 7, **dadurch gekennzeichnet, dass** die Trackingmarker (13, 14, 15 - 63, 64, 65) zum Tracken des Instruments mittels eines medizintechnischen Trackingsystems und zur automatischen Identifizierung des Instruments durch ein medizintechnisches Navigationssystem dienen.

9. Medizinischer Instrumentensatz mit mindestens zwei Instrumenten nach einem der Ansprüche 1 bis 8.

10. Medizinischer Instrumentensatz nach Anspruch 9, bei dem die Instrumente aus verschiedenen ersten Instrumententeilen, die jeweils den funktionellen Abschnitt (11 - 61) umfassen, und aus demselben oder aus gleichen zweiten Instrumententeilen (14 - 64), die mindestens einen Teil des Griffes umfassen, aufgebaut sind.

## Claims

1. A medical instrument (10-60) comprising a handle and a functional portion (11-61), wherein an array of tracking markers (13, 14, 15-63, 64, 65) is arranged on the instrument, **characterised in that** the instrument can be divided into at least two parts and re-assembled, wherein at least one tracking marker is arranged on each part.

2. The medical instrument (10-60) according to claim 1, **characterised in that** a first part (12-62) of the instrument (10-60) comprises the functional portion (11-61), and a second part (14-64) of the instrument (10-60) comprises at least a part of the handle.

3. The medical instrument according to claim 2, **characterised in that** at least one tracking marker (13-63) or group of tracking markers is arranged on the first part of the instrument (10-60), wherein the configuration or arrangement of the tracking marker (13-63) or group of tracking markers is unambiguously assigned to the functional portion (11-61) of the instrument (10-60).

4. The medical instrument (10-60) according to claim 3, **characterised in that** the assignment of the tracking marker (13-63) or group of tracking markers is determined by a characteristic arrangement assigned to the functional part, in particular an axial or radial position on the first instrument part (12-62) relative to the tracking marker geometry of the second instrument part (14-64).

5. The medical instrument (10-60) according to any one of claims 1 to 4, **characterised in that** one tracking marker (13-63) is arranged on the first instrument part (12-62) and one tracking marker is arranged on the second instrument part (14-64).

6. The medical instrument (10-60) according to any one of claims 1 to 4, **characterised in that** one tracking marker (13-63) is arranged on the first instrument part (12-62) and a group of tracking markers, comprising two or more tracking markers, is arranged on the second instrument part (14-64).

7. The medical instrument (10-60) according to any one of claims 1 to 6, **characterised in that** the two instrument parts (12-62; 14-64) can be connected by a non-rotational connection, wherein the connection comprises in particular one or more of the following types of connection:
- a screw thread connection (16, 17) comprising positive-lock or friction-lock engaging means (18) between the first and second instrument part (12-62; 14-64);
- a latch connection (26, 28) comprising positive-lock or friction-lock engaging means (28) between the first and second instrument part (12-62; 14-64);
- a plug connection comprising positive-lock or friction-lock engaging means (28) between the first and second instrument part (12-62; 14-64).

8. The medical instrument (10-60) according to any one of claims 1 to 7, **characterised in that** the tracking markers (13, 14, 15-63, 64, 65) serve for tracking the instrument by means of a medical tracking system and for automatically identifying the instrument using a medical navigation system.

9. A set of medical instruments comprising at least two instruments according to any one of claims 1 to 8.

10. The set of medical instruments according to claim 9, wherein the instruments are constructed from various first instrument parts which each comprise the functional portion (11-61), and from the same second instrument part or identical second instrument parts (14-64) which comprise at least a part of the handle.

## Revendications

1. Instrument médical (10-60) avec une poignée et un segment fonctionnel (1-61), avec un agencement de repères de poursuite (13, 14, 15-63, 64, 65) sur l'instrument, **caractérisé en ce que** l'instrument est divisible en au moins deux parties et peut à nouveau être assemblé, au moins un repère de poursuite étant disposé sur chaque partie.

2. Instrument médical (10-60) selon la revendication 1, **caractérisé en ce qu'**une première partie (12-62) de l'instrument (10-60) comprend le segment fonctionnel (1-61) et une seconde partie (14-64) de l'instrument (10-60) comprend au moins une partie de la poignée.

3. Instrument médical (10-60) selon la revendication 2, **caractérisé en ce que** sur la première partie de l'instrument (10-60) est disposé au moins un repère de poursuite (13-63) ou un groupe de repères de poursuite, le repère de poursuite (13-63) ou le groupe de repères de poursuite étant clairement associé, dans leur forme ou leur disposition, au segment fonctionnel (11-61) de l'instrument (10-60).

4. Instrument médical (10-60) selon la revendication 3, **caractérisé en ce que** l'association du repère de poursuite (13-63) ou du groupe de repères de poursuite est déterminée par une disposition caractéristique associée à la partie fonctionnelle, en particulier par une position axiale ou radiale sur la première partie d'instrument (12-62) par rapport à la géométrie de localisation de la seconde partie d'instrument (14-64).

5. Instrument médical (10-60) selon l'une quelconque des revendications 1 à 4, **caractérisé en ce que** sur la première partie d'instrument (12-62) est disposé un repère de poursuite (13-63) et sur la seconde partie d'instrument (14-64) un repère de poursuite.

6. Instrument médical (10-60) selon l'une quelconque des revendications 1 à 4, **caractérisé en ce que** sur la première partie d'instrument (12-62) est disposé un repère de poursuite (13-63) et sur la seconde partie d'instrument (14-64) un groupe de repères de poursuite avec deux repères de poursuite ou plus.

7. Instrument médical (10-60) selon l'une quelconque des revendications 1 à 6, **caractérisé en ce que** les deux parties d'instrument (12-62 ; 14-64) peuvent être reliées par une liaison solidaire en rotation, la liaison comprenant en particulier un ou plusieurs des types de liaison suivants :
- une liaison filetée (16, 17) avec des moyens de prise (18) par complémentarité de formes ou à friction entre la première et la seconde partie d'instrument (12-62 ; 14-64) ;
- une liaison clipsée (26, 28) avec des moyens de prise (28) par complémentarité de formes ou à friction entre la première et la seconde partie d'instrument (12-62 ; 14-64) ;
- une liaison à enfichage avec des moyens de prise (28) par complémentarité de formes ou à friction entre la première et la seconde partie d'instrument (12-62 ; 14-64).

8. Instrument médical (10-60) selon l'une quelconque des revendications 1 à 7, **caractérisé en ce que** les repères de poursuite (13, 14, 15-63, 64, 65) servent à la localisation de l'instrument au moyen d'un système médical de poursuite et à l'identification automatique de l'instrument par un système médical de navigation.

9. Jeu d'instruments médicaux avec au moins deux instruments selon l'une quelconque des revendications 1 à 8.

10. Jeu d'instruments médicaux selon la revendication 8, dans lequel les instruments sont constitués à partir de différentes premières parties d'instrument, qui comprennent chacune le segment fonctionnel (11-61), et à partir de la même seconde partie d'instrument ou de secondes parties d'instrument identiques (14-64) qui comprennent au moins une partie de la poignée.
